# EUROPÄISCHE PATENTSCHRIFT

(11) **EP 2 800 533 B1**
(45) Veröffentlichungstag und Bekanntmachung des Hinweises auf die Patenterteilung: **10.04.2019**
(21) Anmeldenummer: 12813879.9
(22) Anmeldetag: 27.12.2012
(51) Int. Cl.: A61B 18/14, A61B 17/28

(54) **ELEKTROCHIRURGISCHES INSTRUMENT UND MAULTEIL HIERFÜR**
ELECTROSURGICAL INSTRUMENT AND JAW PART THEREFOR
INSTRUMENT ÉLECTROCHIRURGICAL ET PARTIE DE MÂCHOIRE POUR LEDIT INSTRUMENT

(30) Priorität: 04.01.2012 DE 102012100040
(43) Veröffentlichungstag der Anmeldung: 12.11.2014
(73) Patentinhaber: Aesculap AG, 78532 Tuttlingen (DE)
(72) Erfinder: ROTHWEILER, Christoph, 78166 Donaueschingen (DE); HERNER, Eugen, 72336 Balingen (DE); HUBER, Christian, 78570 Mühlheim (DE)
(74) Vertreter: Winter, Brandl, Fürniss, Hübner, Röss, Kaiser, Polte - Partnerschaft mbB
(86) Internationale Anmeldenummer: PCT/EP2012/076945
(87) Internationale Veröffentlichungsnummer: WO 2013/102602

(56) Entgegenhaltungen:
- EP-A1- 1 878 400
- EP-A1- 2 165 662
- WO-A1-02/080796
- DE-A1-102006 042 985
- DE-A1-102008 008 309
- DE-U1-202012 100 017

## Beschreibung

Die vorliegende Erfindung ist in den Ansprüchen definiert und betrifft ein elektrochirurgisches Instrument, insbesondere für laparoskopische Eingriffe, gemäß dem Oberbegriff des Anspruchs 1.

Nach chirurgischer Entfernung eines Hohlgefäßabschnitts, z.B. bei einer Darmresektion aufgrund eines mit einem Tumor befallenen Darmteils, müssen die zwei Hohlgefäßschnitte an ihren eröffneten Enden wieder so miteinander verbunden werden, dass ein kontinuierlicher Verlauf entsteht. Man spricht hierbei von einer End-zu-End-Anastomose. Standardmäßig werden hierbei die beiden eröffneten Enden z.B. mit Klammernahtgeräten wieder aneinander genäht.

Insbesondere bei Dünn- und Dickdarmeingriffen kommt es zuweilen zu undichten Nahtverbindungen (Nahtinsuffizienz), welche mit einem schweren Krankheitsverlauf und auch einer hohen Sterberate verbunden ist.

Eine Alternative zum Zusammennähen der Hohlgefäßabschnitte stellt die Thermofusions-Technik (TFT) dar. Die Thermofusion mittels Hochfrequenztechnik (HF) beruht auf der Denaturierung von Proteinen, die in vielen Geweben enthalten sind. Hierdurch ist es möglich, kollagenhaltige Gewebe zu verschweißen. Das Gewebe wird während des Schweißvorganges auf Temperaturen oberhalb der

Eiweißdenaturierungstemperatur erhitzt und zusammen mit der intra- und extrazellulären Matrix in einen gelartigen Zustand gebracht. Nach Zusammendrücken der Gewebeflächen kühlt das verflüssigte Gewebe zu einer fusionierten Masse ab, was eine sichere Verbindung der Gewebe bewirkt.

Zum Verschweißen der Hohlgefäßschnitte wird das zwischen zwei Klemmbacken gefasste Gewebe mit einem Strom beaufschlagt, der zwischen Elektroden an den beiden Klemmbacken fließt.

Damit es nicht zum Versagen der Versiegelung bzw. der Verschweißung kommt, müssen die auf das Gewebe einwirkenden Parameter erfasst und geregelt werden. Um dies zu gewährleisten, benötigt man eine genaue Kontrolle von Temperatur, Druck, Gewebeimpedanz, Abstand und Lage.

Es ist wünschenswert, das zwischen den Klemmbacken gehaltene Gewebe gleichmäßig zu behandeln, so dass alle Bereiche zuverlässig erreicht werden und kein Bereich mit einem zu hohen Strom beaufschlagt wird. Dazu muss sichergestellt werden, dass die HF-Elektroden gleichmäßig voneinander beabstandet sind bzw. parallel zueinander ausgerichtet sind.

Aus dem Stand der Technik sind keine Instrumente geeigneter Größenordnung für die Anwendung an den oben genannten Hohlgefäßen und Gewebearten bekannt. Bei Koagulationsinstrumenten kleinerer Bauart, wie z.B. in EP 1 747 762 A2 gezeigt, kommt es, bedingt durch die Bauart, beim Schließen der Klemmbacken zu einer nicht parallelen Ausrichtung der HF-Elektroden.

Der Abstand zwischen den Elektroden kann durch an den Klemmbacken angebrachte Abstandshalter eingehalten werden. Wenn jedoch auf den Klemmbacken eine größere Anzahl von Abstandshaltern vorgesehen ist, wie dies z.B. in EP 1 656 901 B1, EP1 952 777 A1, EP 1 372 507 A1 oder US 2004/122423 A1, DE10 2008 008 309 oder EP 1 878 400 gezeigt ist, perforieren die Abstandshalter zwangsläufig das zu behandelnde Gewebe, da das Gewebe unter den Abstandhaltern bei geschlossenen Klemmbacken derart komprimiert wird, dass es zu dauerhaften Gewebeschädigungen kommt. Dies hat negative Auswirkungen auf das Ergebnis der Versiegelung.

Wenn der Anpressdruck der Klemmbacken reduziert wird, um eine Perforation des Gewebes zu vermeiden, und das Gewebe unter den Abstandshaltern lediglich eingeklemmt wird, führt dies zu einer winkligen Auslenkung der Klemmbacken.

Da die Abstandshalter ferner aus elektrisch nicht leitendem Material sind, um einen Kurzschluss zwischen den HF-Elektroden zu vermeiden, entsteht im Bereich dieser Abstandshalter ein sog. Koagulationsschatten, d.h. dass die Gewebeabschnitte im Bereich der oder unter den Abstandshaltern abgekapselt sind, somit nicht oder nur ungenügend mit Strom beaufschlagt werden und es dort zu keiner zufriedenstellenden Verschweißung der Gefäßabschnitte kommt. Außerdem hat es sich gezeigt, dass derartige, elektrisch nichtleitende Abstandshalter insbesondere dann, wenn sie auf der Elektrode beispielsweise durch Kleben befestigt werden, leicht abplatzen können, um dann in den Patientenkörper ggf. unbemerkt zu gelangen. Darüber hinaus ist dann der vordefinierte Elektrodenabstand nicht mehr gewährleistet.

Vor diesem Hintergrund liegt der vorliegenden Erfindung die Aufgabe zugrunde, ein Instrument bereitzustellen, das mittels Thermofusions-Technik das Ergebnis einer Endzu-End-Anastomose von Hohlgefäßen wie Dünn- und Dickdarm bzw. allgemein bei Gewebeverbindungen verbessert, insbesondere eine parallele Ausrichtung der HF-Elektroden ohne Schädigung des Gewebes sicherstellt sowie eine erhöhte Funktionssicherheit hat.

### Zusammenfassung der Erfindung

Die Aufgabe wird durch ein gattungsgemäßes medizinisches Instrument mit den Merkmalen gemäß dem Anspruch 1 gelöst. Vorteilhafte Weiterbildungen sind Gegenstand der Unteransprüche.

Die vorliegende Erfindung beruht auf der zuvor kurz angedeuteten neuen Erkenntnis, dass auf den Elektrodenflächen unmittelbar angeordnete bzw. darauf fixierte Abstandshalter, welche notwendiger Weise bisher aus einem elektrisch nicht leitenden Material bestehen müssen, den Stromfluss in das eingeklemmte Gewebe lokal einschränken/behindern, sodass keine durchgehende Gewebeschweißnaht gewährleistet ist. Um diese negativen Auswirkungen zu verringern wäre es grundsätzlich möglich, die Abstandshalter möglichst klein zu dimensionieren, z.B. dorn- oder nadelförmig. Es zeigt sich jedoch, dass derart geformte Abstandshalter den Klemmkräften zwischen den Branchen nicht standhalten und daher der Branchenabstand in geschlossener Stellung nicht stabil aufrechterhalten können. Außerdem besteht die Gefahr, dass derart nichtleitende, noppenförmige Vorsprünge in Gebrauch leicht abbrechen können und dann beispielsweise während einer Operation im Gewebe eines Patienten unbemerkt verbleiben. Aus diesem Grund schlägt die vorliegende Erfindung grundsätzlich vor, die auf den Elektrodenflächen unmittelbar angeordneten bzw. darauf fixierten, vorzugsweise noppenförmigen Abstandshalter aus einem elektrisch leitenden Material beispielsweise einstückig/angelötet/angeschweißt mit der betreffenden Elektrode zu fertigen/zu verbinden und statt dessen auf der jeweils gegenüberliegenden Elektrode nichtleitende Abstandshalter-Aufstandsflächen zu schaffen, auf denen die elektrisch leitenden Abstandshalter in Stützeingriff kommen. Da die elektrisch nichtleitenden Abstandshalter-Aufstandsflächen beispielsweise in Form von aufgeklebten, aufgetragenen, eingesetzten oder eingefüllten Pads/Plättchen/Pins im Wesentlichen eben zu der Elektrodenfläche sind, also keinen oder einen nur geringen Überstand zu der betreffenden Elektrodenfläche aufweisen, können diese nicht oder nur schwer von der Elektrodenfläche abgelöst/abgerissen werden. Demnach kann jede der Abstandshalter-Aufstandsflächen gegenüber einem bisher bekannten elektrisch nichtleitenden Abstandshalter hinsichtlich der Flächenmaße kleiner dimensioniert sein, da von der Abstandshalter-Aufstandsfläche infolge geringen bis fehlenden Überstands keine Scherkräfte in die Elektrode eingeleitet werden müssen. Auch die Abstandshalter selbst können aus einem Material wie beispielsweise Metall bestehen, das hohen Scherkräften Stand hält, sodass diese ebenfalls klein dimensioniert sein können. Dies trägt insgesamt dazu bei, Koagulationsschatten zu vermeiden und gleichzeitig die Funktionssicherheit zu erhöhen.

Vorteilhaft ist es, wenn die Abstandshalter-Aufstandsflächen in Form von pads oder pins in entsprechende Vertiefungen oder Ausnehmungen (Mulden) an der Oberfläche der jeweiligen Elektrode eingesetzt sind, sodass diese eine im Wesentlichen ebene Oberfläche mit der Elektrode (ohne Überstand) ausbilden. Auch ist es vorteilhaft, wenn jede Abstandshalter-Aufstandsfläche in Form eines pin ausgebildet ist mit einem flachen Tellerabschnitt, an dessen Unterseite ein Dornfortsatz angefügt ist. Dieser Dornfortsatz steckt in einer entsprechenden Bohrung innerhalb der Elektrode und bewirkt so einen noch festeren Sitz/Halt der Abstandshalter-Aufstandsfläche in der Elektrodenausnehmung.

Zusätzlich zu den vorstehend beschriebenen Maßnahmen kann es vorgesehen sein, die Zahl an auf der jeweiligen Elektrodenfläche fixierten Elektroden und damit an eingesetzten nichtleitenden Abstandshalter-Aufstandsflächen möglichst klein zu halten, um die Auswirkungen von Koagulationsschatten zu reduzieren.

Ein erfindungsgemäßes Koagulationsinstrument für chirurgische Zwecke der einschlägigen Gattung weist demnach im Konkreten gegeneinander (vorzugsweise scheren- oder maulartig) bewegbare Instrumentenbranchen mit jeweils einer oder mehreren Elektrodenflächen an den jeweils zugewandten Branchenseiten auf, zwischen welchen ein Gewebe eingeklemmt und elektrothermisch behandelt werden kann. Die Bewegung der Instrumentenbranchen relativ zueinander wird durch zumindest einen auf proximale Endabschnitte der Instrumentenbranchen wirkenden ersten Abstandshalter bestehend aus einem elektrisch leitenden Material und einer pad- oder pinförmigen Abstandshalter-Aufstandsfläche aus elektrisch nichtleitendem Material sowie zumindest einen auf distale Endabschnitte der Instrumentenbranchen wirkenden zweiten Abstandshalter vorzugsweise aus einem elektrisch nicht leitenden Material begrenzt. Erfindungsgemäß ist auf jeder Elektrodenfläche maximal ein als Abstandshalter wirkender Vorsprung aus elektrisch leitendem Material vorzugsweise einstückig mit der Elektrode beispielsweise durch Prägen oder Stanzen unmittelbar vorgesehen bzw. ausgebildet oder darauf beispielsweise durch Schweißen oder Löten fixiert. Auf der gegenüberliegenden Elektrode ist der pad oder pin aus elektrisch nichtleitendem Material in eine entsprechende Vertiefung eingesetzt oder darauf aufgesetzt/aufgeklebt, um die Abstandshalter-Aufstandsfläche für den einen Vorsprung zu bilden.

Durch das Vorsehen eines Abstandshalters und einer zugehörigen Abstandshalter-Aufstandsfläche, die auf die proximalen Endabschnitte der Instrumentenbranchen wirken, und das Vorsehen eines vorzugsweise elektrisch nicht leitenden Abstandshalters oder Abstandshalter-Aufstandsflächenkombination, der/die auf die distalen Endabschnitte der Instrumentenbranchen wirkt, wird grundsätzlich sichergestellt, dass die Elektroden über ihre gesamte Länge einen vorbestimmten Abstand zueinander haben und somit vorzugsweise parallel zueinander verlaufen. Aufgrund des großen Abstands der Abstandshalter bzw. deren Einwirkstellen in Branchenlängsrichtung wird die Parallelität der Instrumentenbranchen und der daran angebrachten Elektrodenflächen verbessert, da dadurch mögliche Fertigungstoleranzen bei der Ausbildung der Abstandshalter nur noch geringe Auswirkungen auf die Parallelität der Branchen in Schließstellung haben. Durch den so einstellbaren, im Wesentlichen gleichmäßigen Elektrodenabstand zwischen den beiden Branchen in Schließstellung kommt es zu einer gleichmäßigen Durchdringung des Gewebes mit HF-Energie und zu einer gleichmäßigen Stromdichte im Gewebe.

Wie eingangs bereits erwähnt, würde bei einer zu großen Anzahl von Abstandshaltern, insbesondere wenn diese auf den Elektrodenflächen unmittelbar angeordnet/fixiert sind, das zu versiegelnde Gewebe zu stark vorgeschädigt. Ferner entstünden entsprechend viele Koagulationsschatten.

Erfindungsgemäß wird die Schädigung des Gewebes und die inhomogene Durchdringung des Gewebes mit HF-Energie dadurch weiter minimiert, dass auf jeder Elektrodenfläche maximal ein Abstandshalter aufgebracht ist, was auch bedeutet, dass ausgewählte Elektrodenflächen überhaupt keinen einzigen Abstandshalter in Form eines darauf fixierten oder daran ausgeformten (noppenförmigen) Vorsprungs (aus elektrisch leitendem Material) aufweisen und der/die Abstandshalter stattdessen an anderer Stelle (d.h. außerhalb der Elektrodenflächen) auf der Instrumentenbranche oder mit anderen Mitteln (z.B. nicht fixiert sondern lose) realisiert wird, wodurch das Gewebe weniger geschädigt und die Ausbildung von Koagulationsschatten weiter reduziert wird.

Die erfindungsgemäße Koagulationsklemme bzw. die erfindungsgemäßen Instrumentenbranchen einer solchen Koagulationsklemme schaffen somit einen optimalen Kompromiss zwischen maximaler paralleler Ausrichtung der HF-Elektroden in Schließstellung der Branchen einerseits und homogener Gewebefusion bei minimaler Gewebeschädigung anderseits. Daher werden durch die Abstandshalter verursachte Gewebeschäden in Folge überhöhter Krafteinwirkung auf das eingespannte Gewebe verhindert und eine sichere Fusion der einzelnen Gewebebestandteile durch gleich bleibende Kraftverhältnisse, durch die parallele Anordnung der Elektroden, durch den klar definierten Abstand der Elektroden und durch die homogene Stromverteilung im Gewebe längs der Elektroden gewährleistet. Außerdem werden durch die spezielle Anordnung zumindest einer Teilanzahl an Abstandshaltern außerhalb der Elektrodenflächen Kurzschlüsse zwischen den Elektroden und Leckagen an den versiegelten Gewebeschichten vermieden, auch dann, wenn diese speziellen Abstandshalter aus einem leitenden Material bestehen. Dadurch werden gleich bleibende Voraussetzungen für die HF-Chirurgie insbesondere im Hinblick auf die Gewebeimpedanz geschaffen, sodass die Qualität der versiegelten Gewebebereiche besser elektrisch kontrolliert werden kann.

Gemäß einem anderen oder zusätzlichen Aspekt der Erfindung sind die (alle) Abstandshalter ausschließlich außerhalb eines für die Behandlung des Gewebes vorgesehenen Bereichs vorzugsweise nur am proximalen und distalen Ende der Branchen angeordnet. Wenn die Abstandshalter lediglich auf die proximalen und distalen Endabschnitte der Instrumentenbranchen wirken und in dem medialen Bereich der Instrumentenbranchen, der in der Regel den eigentlichen bzw. wesentlichen Behandlungsbereich des Gewebes darstellt, frei von Abstandshaltern ist, werden jegliche Schädigung des Gewebes durch die Abstandshalter und die durch die Abstandshalter verursachten Koagulationsschatten in diesem Hauptbereich vermieden.

Gemäß einem weiteren oder anderen Aspekt der vorliegenden Erfindung ist ein Abstandshalter, z.B. der auf die proximalen Endabschnitte der Instrumentenbranchen wirkende Abstandshalter, ein separat von den Instrumentenbranchen ausgebildetes Abstandshaltermodul, das zumindest eine elektrisch nichtleitende Materialzunge aufweist, welche in einer Schließstellung der Instrumentenbranchen zwischen diesen einklemmt wird. Die Höhe der Materialzunge entspricht daher einem vorbestimmten einzustellenden (Parallel-) Abstand zwischen den Instrumentenbranchen.

Ein separates Abstandshaltermodul dieser Bauart hat mehrere Vorteile. Zum einen lässt sich dieses auf einfache Weise und unabhängig von den jeweiligen Instrumentenbranchen bzw. der Koagulationsklemme, für die es verwendet werden soll, herstellen. Zum anderen kann dieses jederzeit ausgetauscht werden, sei es aus Verschleißgründen oder zum Ersetzen durch ein anderes Abstandshaltermodul mit höheren oder niedrigeren Materialzungen. Dadurch kann der Abstand der Instrumentenbranchen in der Schließstellung variiert werden. Die körperliche Trennung von Instrumentenbranchen und Abstandshaltern hat somit den Vorteil, dass dasselbe Abstandshaltermodul für unterschiedliche Instrumentenbranchen oder dass für dieselben Instrumentenbranchen unterschiedliche Abstandshaltermodule bereitgestellt werden können. Auch zeigt sich der Koagulationsschatteneffekt im Fall einer lose gehaltenen sowie zwischen den Elektrodenflächen in Schließstellung der Branchen eingespannten Materialzunge geringer, als im Fall eines auf den Elektrodenflächen fixierten Abstandshalters, auch dann wenn der Abstandshalter aus elektrisch leitendem Material besteht und mit einer Abstandshalter-Aufstandsfläche aus elektrisch nicht leitendem Material zusammenwirkt.

Das Abstandshaltermodul kann mehrere seitlich bzw. in Querrichtung der Branchen voneinander beabstandete Materialzungen (aus nicht leitendem Material) aufweisen, um z.B. einen zwischen zwei Koagulationselektrodenflächen vorgesehenen elektrischen Schneideabschnitt auszusparen.

Zum Öffnen und Schließen der Instrumentenbranchen kann zumindest eine der Instrumentenbranchen schwenkbar beispielsweise an einem Instrumentenschaft oder an der gegenüberliegenden Branche gelagert und über einen (im Instrumentenschaft und/oder im Griffstück gelagerten) Handhabungsmechanismus betätigbar sein, um die Instrumentenbranchen gegen- und voneinander zu bewegen. Das Abstandshaltermodul kann dabei drehbar in einem Schwenkgelenk der betätigbaren (gelagerten) Instrumentenbranche gelagert sein, insbesondere dabei von Gelenkabschnitten der betätigbaren Instrumentenbranche gehäuseartig umgriffen sein.

Durch die Integration des Abstandhaltermoduls in das Schwenkgelenk einer oder beider Instrumentenbranchen ist dieses nicht nur platzsparend im Inneren des Instruments bzw. des Maulteils untergebracht, sondern übt seine Abstandshalterfunktion ohne zusätzliche Betätigung durch den Chirurgen von selbst aus, wenn die Instrumentenbranchen geschlossen werden.

Alternativ oder zusätzlich zu dem oben erwähnten separaten Abstandshaltermodul kann zumindest eine der Instrumentenbranchen, vorzugsweise die schwenkbare Branche einen in einer Kulisse auf Seiten der anderen Branche geführten Drehbegrenzungsstift aufweisen, wobei das Zusammenspiel von Drehbegrenzungsstift und Kulisse eine Art Abstandshalter, insbesondere den auf die proximalen Endabschnitte der Instrumentenbranchen wirkenden Abstandshalter simuliert bzw. bildet und die Instrumentenbranchen zueinander einen vorbestimmten Abstand einnehmen, wenn der zumindest eine Drehbegrenzungsstift einen Endabschnitt der Kulisse erreicht. Für den Fall, dass beide Instrumentenbranchen schwenkbar oder anderweitig bewegbar (z.B. verschiebbar) gelagert sind, kann der Freiheitsgrad beider Instrumentenbranchen jeweils durch einen in einer jeweiligen Kulisse geführten Drehbegrenzungsstift begrenzt werden.

Diese Lösung hat insbesondere den Vorteil, dass der Abstandshalter zumindest im proximalen Endabschnitt der Branchen vollständig außerhalb des Klemmbereichs der Instrumentenbranchen, d.h. außerhalb des Gewebebehandlungsbereichs (Elektrodenflächen) angeordnet werden kann und es zu keinerlei Kontakt zwischen Abstandshalter und dem zu behandelnden Gewebe kommt. Auf diese Weise kann eine Schädigung des Gewebes sicher verhindert werden.

Ein Abstandshalter, z.B. der auf die distalen Endabschnitte der Instrumentenbranchen wirkende Abstandhalter, kann durch einen zwischen zwei Elektrodenflächen angeordneten und zur anderen Instrumentenbranche hin weisenden (noppenförmigen) Vorsprung gebildet werden. Wenn der Abstandshalter demzufolge nicht auf den Elektrodenflächen angeordnet ist, sondern daneben oder dazwischen, entstehen in diesem Bereich keine Koagulationsschatten, insbesondere auch deshalb, weil dann der/die Abstandshalter nicht aus isolierendem Material sein muss/müssen. Wenn der Abstandshalter zwischen den Elektrodenflächen angeordnet ist, insbesondere wenn er auf der Mittelachse einer der Instrumentenbranchen angeordnet ist und zwar ohne direkten Kontakt mit den Elektrodenflächen, kommt es zu keinem elektrischen Kurzschluss zwischen den Elektrodenflächen. Außerdem ergibt sich keine Torsionsbeanspruchung der Instrumentenbranchen, wenn diese in der Schließstellung gegeneinander gepresst und lediglich durch den Abstandshalter auseinander gehalten werden. Dadurch kann die Anzahl der Abstandshalter insgesamt weiter reduziert werden.

Alternativ oder zusätzlich kann ein oder mehrere Abstandshalter, insbesondere die auf die proximalen und/oder distalen Endabschnitte der Instrumentenbranchen einwirkenden Abstandshalter durch nur einen auf einer Elektrodenfläche oder durch mehrere auf jeweils unterschiedlichen Elektrodenflächen unmittelbar vorgesehene/fixierte Vorsprünge gebildet werden. Dadurch wird einerseits eine parallele Ausrichtung der Elektroden in Längsrichtung sichergestellt und andererseits die Anzahl der Abstandshalter fixiert auf den Elektrodenflächen gering gehalten, wodurch eine optimale Behandlung, insbesondere eine homogene Fusion des Gewebes ermöglicht wird.

Insbesondere bei besonders langen Instrumentenbranchen oder solchen, die nach Art einer Wippe mittig beispielsweise am Ende eines Instrumentenschafts gelagert sind, muss sichergestellt werden, dass die Elektrodenflächen im medialen Bereich den gewünschten Abstand halten und dieser aufgrund von Biegebeanspruchung (dann nämlich, wenn die beiden Branchen an ihren Endabschnitten aufeinander gepresst werden) nicht unterschritten wird. Deshalb kann neben dem ersten und zweiten Abstandshalter, der auf proximale bzw. distale Endabschnitte der Instrumentenbranchen wirkt, zumindest ein dritter Abstandshalter gemäß der Erfindung vorgesehen sein, der auf mediale Abschnitte der Instrumentenbranchen wirkt, wobei die zumindest drei Abstandshalter grundsätzlich so angeordnet sind, dass auf jeder Elektrodenfläche einer Branche maximal nur ein Abstandshalter fixiert/ausgebildet ist. Im konkreten Fall wäre demnach der dritte Abstandshalter auf einer anderen Elektrodenfläche als der erste und/oder zweite Abstandshalter oder zwischen den Elektrodenflächen angeordnet. Auch wenn in diesem Fall der medial wirkende Abstandshalter in Kontakt mit dem Gewebe kommen kann, so wird dennoch durch die spezifische Anordnung und Anzahl der Abstandshalter gemäß vorstehender Definition dafür Sorge getragen, dass die Auswirkungen auf das Gewebe einerseits minimal gehalten werden und andererseits ein gleichmäßiger Abstand zwischen den Elektroden über die gesamt Länge der HF-Elektroden, bzw. der Branchen sichergestellt wird.

Zwischen dem proximalen und dem distalen Endabschnitt zumindest einer Instrumentenbranche können zusätzlich ein oder mehrere zur anderen Instrumentenbranche hin weisende (noppenförmige) Erhöhungen ausgebildet sind, deren Höhe geringer als die Höhe der Abstandshalter ist, insbesondere 10% bis 75% der Höhe der Abstandhalter beträgt.

Durch die zusätzlich auf der Instrumentenbranche, insbesondere auf einer oder beiden Elektrodenflächen einer oder beider Branchen, angebrachten Erhöhungen oder Zähne kann das Gewebe besser gehalten werden, um zu verhindern, dass das zu behandelnde Gewebe oder die zu behandelnden Gewebeabschnitte aus den Instrumentenbranchen herausrutschen, bevor diese miteinander fusioniert sind.

Da diese Erhöhung niedriger als der durch die Abstandshalter definierte Minimalabstand der beiden Instrumentenbranchen in der Schließstellung ist, z.B. 10% - 75% des Abstands, kommen diese nie in Anlage mit der gegenüberliegende Instrumentenbranche. Deshalb wird das Gewebe zwischen der Erhöhung und der gegenüberliegenden Instrumentenbranche nicht perforiert und somit nicht dauerhaft geschädigt. Ferner entstehen durch diese Erhöhungen auch keine Koagulationsschatten, da das zwischen der Erhöhung und der gegenüberliegenden Instrumentenbranche eingeklemmte Gewebe nicht wesentlich abdeckt wird. Die Koagulationsschatten, die durch die herkömmlichen Instrumente entstehen, werden so vermieden. Da diese Erhöhungen ohnehin nicht in Anlage mit der gegenüberliegenden Instrumentenbranche kommen, können diese ebenfalls auch aus elektrisch leitendem Material sein, wobei in diesem Fall auf gegenüberliegende pads/pins aus elektrisch nichtleitendem Material verzichtet werden kann. Es können natürlich pro Elektrodenfläche mehrere solcher Erhöhungen angeordnet sein. Diese können in gleichmäßigen Abständen angeordnet sein.

Wie bereits vorstehend zum Teil angedeutet wurde, können die Instrumentenbranchen zwei oder mehrere einander gegenüberliegende, z.B. parallel verlaufende, Elektrodenflächenpaare aufweisen, wobei die Vorsprünge und/oder Erhöhungen an unterschiedlichen Elektrodenflächenpaaren ausgebildet sind. Insbesondere die nicht betätigbare Instrumentenbranche kann etwa mittig und zu einem gewissen Grad nach Art einer Wippe schwenkbar beispielsweise am Endabschnitt eines Instrumentenschafts gelagert sein, um etwaige Winkelabweichungen bezüglich der betätigbaren Instrumentenbranche in Schließstellung ausgleichen zu können. Selbstverständlich kann die Koagulationsklemme bzw. das Maulteil auch zwei voneinander trennbare oder zwei zueinander translatorisch verschiebbare Branchen aufweisen.

Es ist zu beachten, dass die zuvor genannten Aspekte und Merkmale sowohl einzeln, als auch zu mehreren miteinander kombiniert werden können.

### Kurze Beschreibung der Zeichnungen

Fig. 1 zeigt ein elektrochirurgisches Instrument gemäß einer ersten Ausführungsform der Erfindung;
Fig. 2 zeigt eine perspektivische Ansicht zweier (scherenförmig) zueinander verschwenkbarer Instrumentenbranchen des elektrochirurgischen Instruments gemäß der ersten Ausführungsform der Erfindung;
Fig. 3 zeigt eine vergrößerte Ansicht eines Abstandhaltermoduls gemäß der ersten Ausführungsform der Erfindung;
Fig. 4 zeigt eine Seitenansicht der beiden Instrumentenbranchen der Fig. 2 in einer geöffneten Stellung;
Fig. 5 zeigt eine Seitenansicht der beiden Instrumentenbranchen der Fig. 2 in einer geschlossenen Stellung;
Figuren 6A, 6B, 6C zeigen Detailansichten A, B bzw. C der Fig. 4 bzw.5.
Fig. 7 zeigt eine perspektivische Ansicht zweier zueinander verschwenkbarer Instrumentenbranchen eines elektrochirurgischen Instruments gemäß einer zweiten Ausführungsform der Erfindung;
Fig. 8 zeigt eine perspektivische Ansicht zweier zueinander verschwenkbarer Instrumentenbranchen eines elektrochirurgischen Instruments gemäß einer dritten Ausführungsform der Erfindung;
Fig. 9 zeigt eine Seitenansicht der beiden Instrumentenbranchen der Fig. 8 in einer geöffneten Stellung;
Figuren 10A, 10B und 10C zeigt Detailansichten A, B bzw. C der Fig. 9;
Fig. 11 zeigt eine Seitenansicht der beiden Instrumentenbranchen der Fig. 8 in einer geschlossenen Stellung;
Figuren 12A, 12B, 12C und 12D zeigen Detailansichten A, B, C bzw. D der Fig. 11;
Fig. 13 zeigt eine Seitenansicht zweier zueinander verschwenkbarer Instrumentenbranchen eines elektrochirurgischen Instruments gemäß einer vierten Ausführungsform der Erfindung;
Fig. 14 zeigt eine perspektivische Ansicht zweier zueinander verschwenkbarer Instrumentenbranchen des elektrochirurgischen Instruments gemäß der vierten Ausführungsform der Erfindung;
Fig. 15 zeigt eine perspektivische Ansicht zweier zueinander verschwenkbarer Instrumentenbranchen eines elektrochirurgischen Instruments gemäß einer fünften Ausführungsform der Erfindung;
Fig. 16 zeigt ein elektrochirurgisches Instrument gemäß einer sechsten Ausführungsform der Erfindung;
Fig. 17 zeigt ein elektrochirurgisches Instrument gemäß einer siebten Ausführungsform der Erfindung;
Fig. 18 zeigt die Detailansicht eines Abstandshalters sowie einer Abstandshalter-Aufstandsfläche gemäß einem ersten Ausführungsbeispiel der Erfindung;
Fig. 19 zeigt die Detailansicht eines Abstandshalters sowie einer Abstandshalter-Aufstandsfläche gemäß einem zweiten Ausführungsbeispiel der Erfindung und
Fig. 20 zeigt Detailansicht eines Abstandshalters sowie einer Abstandshalter-Aufstandsfläche gemäß einem dritten Ausführungsbeispiel der Erfindung.

### Detaillierte Beschreibung bevorzugter Ausführungsformen

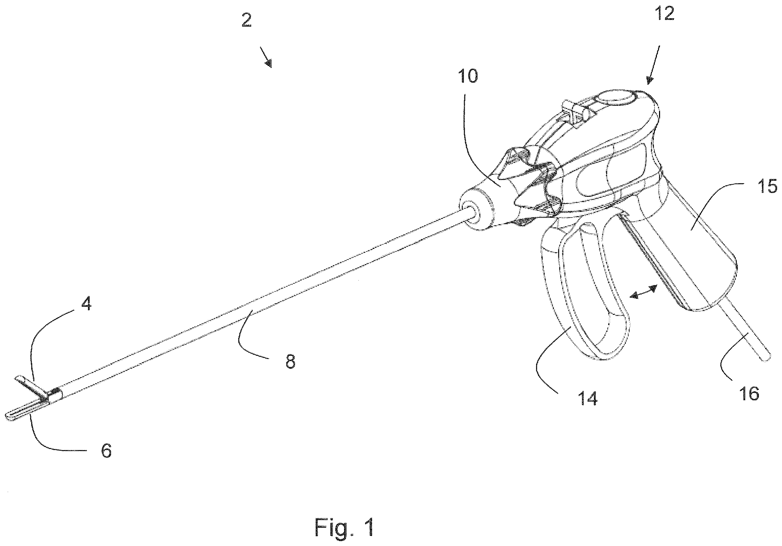

Fig. 1 zeigt eine perspektivische Ansicht eines laparoskopischen elektrochirurgischen Instruments 2 gemäß einer ersten Ausführungsform der Erfindung mit einem Maulteil bestehend aus einem Paar vorzugsweise scheren- oder zangenartig zueinander sich bewegender Instrumentenbranchen 4 und 6 in einer geöffneten Stellung, die am distalen Ende eines Instrumentenschafts 8 angeordnet sind, der wiederum über eine manuell betätigbare Schaftdreheinrichtung 10 an einem Griffstück oder Handhabungsteil 12 drehbar befestigt ist. Über die Schaftdreheinrichtung 10 kann der Schaft 8 und die daran angeordneten Instrumentenbranchen 4 und 6 relativ zum Handhabungsteil 12 um die Schaftlängsachse verdreht werden. Das Handhabungsteil 12 weist einen manuell betätigbaren Handgriff oder Abzugsbügel 14 auf, der relativ zu einem mit dem Handhabungsteil 12 fest verbundenen Hand- oder Pistolengriff 15 schwenkend bewegbar ist. Die Instrumentenbranchen 4, 6 oder zumindest eine manuell betätigbare Instrumentenbranche 4 stehen über einen (nicht weiter gezeigten) Betätigungsmechanismus, z.B. einem Seilzug oder einer Schubstange innerhalb des Instrumentenschafts 8, in Wirkverbindung mit dem Handgriff 14 und können durch manuelle Betätigung des Handgriffs 14 vorzugsweise stufenlos von einer geöffneten in eine geschlossene Stellung (und umgekehrt) gebracht werden. Über eine (nur zum Teil gezeigte) Leitung oder elektrische Verkabelung 16 ist das Handhabungsteil 12 mit einer (nicht gezeigten) HF-Energiequelle verbunden, um zur elektrothermischen Behandlung von Gewebe zwischen den Instrumentenbranchen 4 und 6 eine HF-Spannung anlegen zu können.

Hinsichtlich der grundsätzlichen Funktionsweise und des mechanischen Aufbaus des Instruments 2 insbesondere des Betätigungsmechanismus wird beispielsweise auf die veröffentlichte Druckschrift WO 2011/097469 A2 verwiesen.

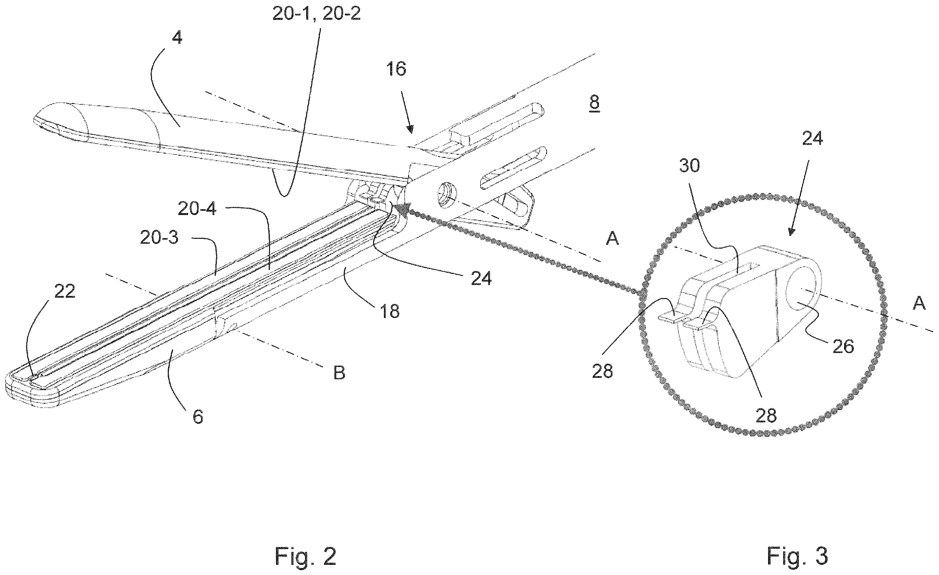

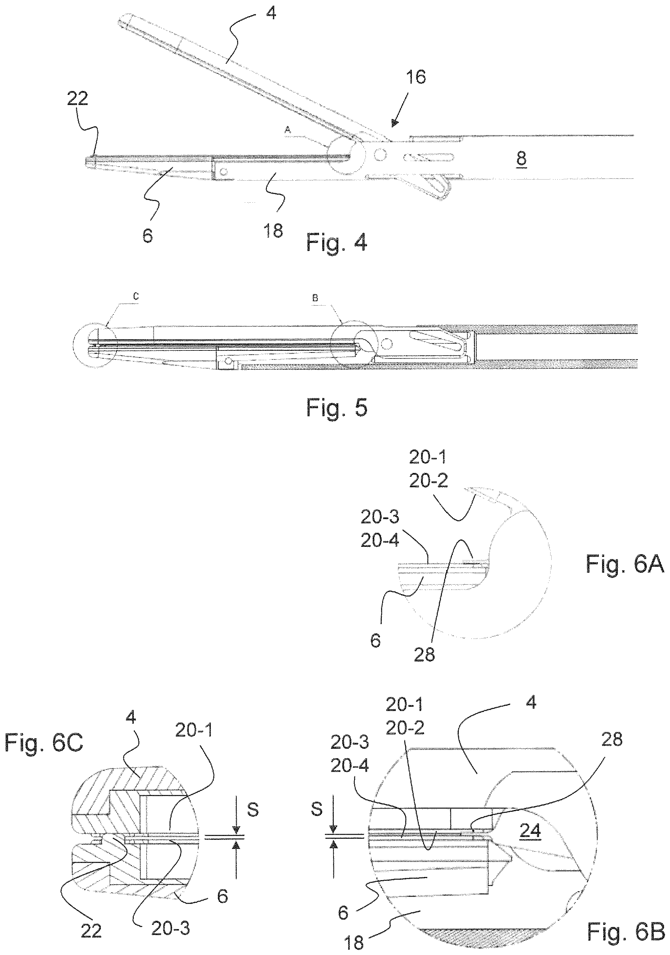

Fig. 2 und 4 zeigen nunmehr das distale Ende des Schafts 8 bzw. das an den Schaft 8 angeschlossene Maulteil mit den Instrumentenbranchen 4 und 6 in einer geöffneten Stellung im Detail. Die erste, gemäß Fig. 2 oder 4 obere Instrumentenbranche 4 ist über ein proximales Schwenkgelenk oder Scharnier 16 (siehe Fig. 4) um eine Querachse A schwenkbar am distalen Ende des Schafts 12 gelagert. Das distale Schaftende oder daran angeschlossene Maulteil hat hierfür einen längs des Schafts sich erstreckenden, mittig ausgebildeten Durchgangsschlitz oder Längsspalt 17, dessen Seitenwangen jeweils eine (co-axial zueinander ausgerichtete) Querbohrung aufweisen, welche die vorstehend genannte Querachse A definieren. Der Durchgangsschlitz 17 hat ferner eine Schlitzbreite, die das beweg-/ schwenkbare Einsetzten der ersten Instrumentenbranche 4 darin erlaubt. In axialer, distaler Verlängerung (Auskragung) zu dem Durchgangsschlitz 17 bildet das distale Schaftende bzw. Maulteil einen halbschalen- oder rinnenförmigen Stützvorsprung oder Träger 18, welcher der drehbaren oberen Instrumentenbranche gegenüber liegt und an seinem distalen Endabschnitt eine Durchgangsquerbohrung, im Wesentlichen parallel zur Querachse A aufweist.

Die zweite, gemäß Fig. 2 oder 4 untere Instrumentenbranche 6 ist, in Schaftlängsrichtung gesehen, nur über einen Teillängenabschnitt in dem schalenförmigen Stützvorsprung 18 (axial) aufgenommen, derart, dass sie über deren verbleibenden Teillängenabschnitt axial über den Stützvorsprung 18 in distaler Richtung vorragt. Des Weiteren ist die untere Instrumentenbranche 6 mittig nach Art einer Wippe schwenkbar an dem Stützvorsprung 18 über die distale Durchgangsquerbohrung angelenkt. Mittels eines nicht gezeigten Federmechanismus (siehe u.a. auch WO 2011/097469 A2) ist der vordere bzw. distale Wippenteil der unteren Instrumentenbranche 6 gemäß Fig. 4 nach oben bzw. zur oberen Instrumentenbranche 4 hin vorgespannt, wodurch die untere Branche 6 in Längsrichtung gesehen einen geringen Winkel zum Instrumentenschaft 8 bzw. zum Stützvorsprung 18 einnimmt und dadurch im Fall eines Zusammenklappens bzw. Schließens des Maulteils die distalen Endabschnitte der beiden Branchen 4, 6 pinzettenartig zuerst in Klemmkontakt kommen. Dadurch wird das Greifen von Gewebe erleichtert. Die untere Instrumentenbranche 6 ist nur soweit am Abschnitt bzw. Stützvorsprung 18 wippenförmig schwenkbar um die von der Durchgangsquerbohrung definierten Achse B gehalten, um kleinere Winkelabweichungen zwischen der oberen und unteren Instrumentenbranche 4 und 6 in Schließstellung ausgleichen zu können, bzw. um eine parallele Ausrichtung beider Branchen 4, 6 zu erreichen.

Jede der Instrumentenbranchen 4 und 6 weist gemäß dem vorliegenden Ausführungsbeispiel bevorzugt zwei in Branchenquerrichtung voneinander beabstandete, in Branchenlängsrichtung im Wesentlichen parallel verlaufende Elektroden oder Elektrodenflächen 20 (20-1, 20-2, 20-3 und 20-4) auf, welche mit HF-Spannung beaufschlagt werden können. Befindet sich demzufolge Gewebe zwischen den Instrumentenbranchen 4 und 6 in ihrer geschlossenen Stellung, kann der Chirurg dieses mit den Elektrodenflächen 20 koagulieren, trennen oder verschweißen. Zwischen den Elektrodenflächen 20 kann zudem ein (nicht gezeigtes) spezielles elektrochirurgisches Messer oder eine entsprechende Schneideeinrichtung angeordnet sein, die von den Elektrodenflächen 20 elektrisch isoliert ist.

Um einen Kurzschluss zwischen den Elektrodenflächen 20 der beiden Instrumentenbranchen 4 und 6 zu vermeiden bzw. um sicherzustellen, dass ein homogener Strom über das zwischen den Elektrodenflächen 20 eingeklemmte Gewebe über die gesamte Elektrodenlänge fließt, müssen die Elektrodenflächen 20 auch in der geschlossenen Stellung voneinander im Wesentlichen gleichmäßig beabstandet bleiben. Das Instrument 2 hat deshalb am distalen Endabschnitt der unteren Instrumentenbranche 6 (und/oder der oberen Instrumentenbranche 4) zwischen den beiden Elektrodenflächen 20-3 und 20-4 einen über die Elektrodenflächen 20-3 und 20-4 um ein vorbestimmtes und dem gewünschten Abstand zwischen den Elektrodenflächen 20 entsprechenden Maß hinaus vorragenden, vorzugsweise noppenförmigen Vorsprung 22, der mit der oberen Instrumentenbranche 4 (und/oder der unteren Instrumentenbranche 6) bei Schließen des Maulteils in Anlage kommt und so als Abstandshalter an den distalen Endabschnitten der beiden Instrumentenbranchen 4 und 6 dient. An den proximalen Endabschnitten der beiden Instrumentenbranchen 4 und 6 wird der Abstand zwischen den Elektrodenflächen 20 gemäß diesem Ausführungsbeispiel durch ein separates, d.h. getrennt von den Branchen 4, 6 bzw. den Elektroden 20 frei gehaltenes Abstandshaltermodul 24 bewerkstelligt. Dieses Abstandshaltermodul 24 ist vorliegend ein nockenförmiges Bauteil mit einem proximalen Lagerungsabschnitt (Nockenabschnitt mit Durchgangsquerbohrung), das mit dem Schwenkgelenk (Schwenbolzen) 16 in Eingriff bringbar ist und das sich zwischen den Instrumentenbranchen 4 und 6 somit frei um die Schwenkachse A drehen kann. Aus Platzgründen ist vorliegend die bewegbare obere (und/oder die untere) Branche 4 an ihrem proximalen Endabschnitt im Bereich der Schwenkachse A in Längsrichtung ausgehöhlt, wodurch sich eine Art Aufnahmeraum oder Längsnut ergibt, der Abmessungen für ein darin Aufnehmen des Abstandshaltermoduls 24 ausreichend sind. D.h. zumindest in Schließstellung des Maulteils ist das Abstandshaltermodul 24 zwischen zwei Nutenwangen zumindest der einen betätigbaren Instrumentenbranche 4 aufgenommen.

Fig. 3 zeigt eine vergrößerte perspektivische Ansicht des Abstandshaltermoduls 24 alleine. Wie vorstehend bereits angedeutet wurde, hat das Modul 24 eine Art eine Nockenform mit dem proximalen Lagerungsabschnitt, in welchem die Nocke eine solche Nockendicke/Höhe hat, das diese beweglich in der proximalen Längsnut der einen Branche 4 versenkbar ist. Im Lagerungsabschnitt des Moduls 24 ist ferner die Querdurchgangsbohrung 26 ausgebildet. Auf der dem Lagerungabschnitt gegenüberliegenden distalen Außenquerseite des Moduls 24 sind zwei sich radial bezüglich der Schwenkachse A vorspringende flache Materialzungen 28 angeformt, deren jeweilige Flachseiten den Branchen 4, 6 zugewandt sind und deren Zungendicke/Höhe H einem zu erzielenden Mindestabstand S (Spaltmaß) zwischen den gegenüberliegenden Elektrodenflächen 20-1 und 20-3 bzw. 20-2 und 20-4 in Schließstellung der Branchen 4, 6 entspricht und deren seitlicher Abstand (in Branchenquerrichtung) und Breite im Wesentlichen dem Parallellabstand und der Breite der Elektrodenflächen 20 entspricht, sodass die Materialzungen 28 auf den Elektrodenflächen zumindest teilweise zu liegen kommen. Zwischen den beiden Materialzungen 28 ist in dem nockenförmigen Modul 24 ein am distalen Ende des Moduls 24 offener Längsschlitz 30 ausgeformt, der bis hin zum Lagerabschnitt erstreckt und unmittelbar vor der Querbohrung 26 endet. Das gesamte Abstandshaltermodul 24 oder zumindest die Materialzungen 28 sind in diesem Ausführungsbeispiel aus elektrisch nicht leitendem Material hergestellt.

Die Figuren 4 und 5 zeigen eine Seitenansicht des Maulteils bzw. der Instrumentenbranchen 4 und 6 einmal in geöffneter und einmal in geschlossener Stellung. Die Figuren 6A, 6B und 6C zeigen Detailansichten des Maulteils gemäß der Figuren 4 und 5.

In der Fig. 6A ist zu erkennen, dass die Materialzungen 28 des Abstandshaltermoduls 24 auf einem proximalen Endabschnitt der Elektrodenflächen 20-3 und 20-4 der unteren Instrumentenbranche 4 lose aufliegen, wenn sich das Maulteil in Offenstellung befindet. Wenn die Instrumentenbranchen 4 und 6 durch Betätigung vorzugsweise der oberen Instrumentenbranche 4 in die geschlossene Stellung gebracht (siehe Fig. 5) wird, werden die Materialzungen 28 des separaten Abstandshaltermoduls 24 zwischen den proximalen Endabschnitten der Elektrodenflächen 20 der beiden Instrumentenbranchen 4 und 6 eingeklemmt (siehe Fig. 6B) und der Vorsprung 22 am distalen Endabschnitt der unteren Instrumentenbranche 6 kommt in Anlage mit dem distalen Endabschnitt der oberen Instrumentenbranche 4. Somit bleiben die proximalen und distalen Endabschnitte und damit auch die gesamten Elektrodenflächen 20 um das vorbestimmte Spaltmaß S voneinander und im Wesentlichen parallel zueinander beanstandet.

Wie vorstehend ausgeführt wurde, ist der (noppenförmige) Vorsprung 22 zwischen den Elektroden angeordnet und kommt somit direkt mit der oberen, betätigbaren Branche 4 (und nicht mit den oberen Elektrodenflächen der Branche 4) in Anlage. Außerdem sind die proximalen Materialzungen 24 nicht direkt auf den Elektrodenflächen fixiert sondern liegen an diesen nur an. Bei der ersten Ausführungsform befindet sich somit kein Abstandshalter unmittelbar auf einer der Elektrodenflächen 20 (im Sinne von darauf fixiert). Damit können Koagulationsschatteneffekte gegenüber dem Stand der Technik verringert werden.

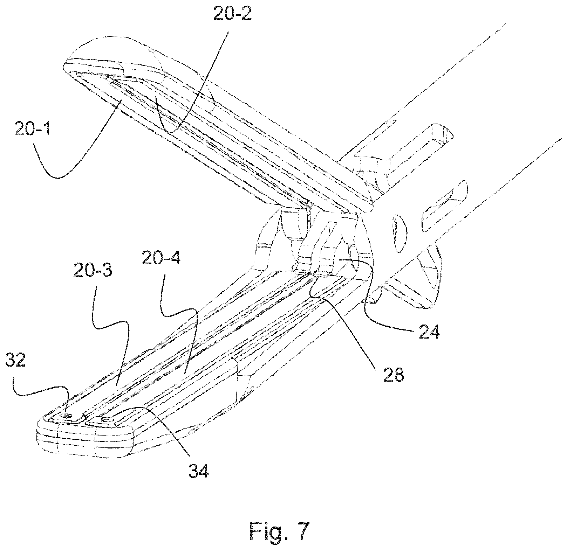

Fig. 7 zeigt eine zweite Ausführungsform, die sich von dem laparoskopischen elektrochirurgischen Instrument 2 der ersten Ausführungsform lediglich in der Anordnung des distalen Abstandshalters unterscheidet, weshalb im Folgenden lediglich auf die Unterschiede zum ersten Ausführungsbeispiel eingegangen wird.

Bei der zweiten Ausführungsform befinden sich anstelle des zwischen den Elektrodenflächen 20-3 und 20-4 angeordneten Vorsprungs 22 zwei (noppenförmige) Vorsprünge 32, 34 unmittelbar (d.h. fest fixiert) auf den distalen Endabschnitten der Elektrodenflächen 20-3 und 20-4. Um einen Kurzschluss zwischen den Elektrodenflächen zu vermeiden, wenn diese in der geschlossenen Stellung nahe zu den Elektrodenflächen der oberen Instrumentenbranche 4 kommen, sind die Vorsprünge 32, 34 aus einem elektrisch leitenden Material gefertigt und kommen mit einer Abstandshalter-Aufstandsfläche aus einem nicht leitenden Material in Anlage, das auf der jeweils gegenüberliegenden Elektrode (ausschließlich) im Bereich des jeweiligen Vorsprungs (punkt-/pad-förmig) vorgesehen ist. Die Vorsprünge 32, 34 können aufgelötet, aufgespritzt oder einstückig (etwa durch Prägen oder Stanzen) ausgebildet sein. Die Abstandshalter-Aufstandsfläche kann durch Aufspritzen, Auftragen, Einfüllen einer aushärtenden Masse oder durch Aufkleben oder Einsetzen eines Isolationsplättchens/-pads/-pins vorzugsweise in eine Vertiefung in der jeweiligen Elektrode ausgebildet sein, wie dies nachfolgend noch näher beschrieben wird.

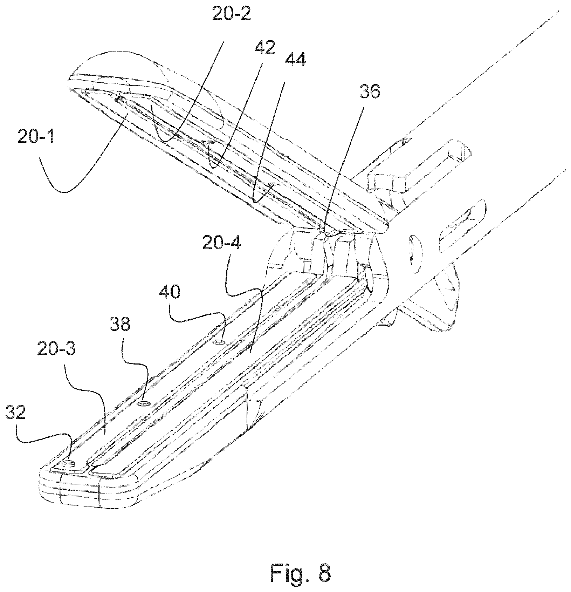

Fig. 8 zeigt eine perspektivische Ansicht eines laparoskopischen elektrochirurgischen Instruments 2 gemäß einer dritten Ausführungsform, die sich von der ersten bzw. zweiten Ausführungsform darin unterscheidet, dass am distalen Abschnitt der unteren (nicht betätigbaren) Instrumentenbranche oder alternative an der oberen, betätigbaren Instrumentenbranche lediglich ein (noppenförmiger) Vorsprung 32 auf einer (20-3) der beiden Elektrodenflächen 20-3 und 20-4 vorgesehen (fixiert) ist und anstelle des bisher verwendeten separaten Abstandshaltermoduls 24 am proximalen Endabschnitt auf einer (20-2) der beiden Elektrodenflächen 20-1 und 20-2 der oberen Instrumentenbranche 4 oder entsprechend alternativ auf der unteren Branche ein (noppenförmiger) Vorsprung 36 angeordnet (fixiert) ist. Wenn die beiden Instrumentenbranchen 4 und 6 geschlossen werden, kommt der Vorsprung 32, fixiert auf der unteren Elektrodenfläche 20-3, in Anlage mit der oberen Elektrodenfläche 20-1, d.h. dem Isolationsplättchen an der oberen Elektrode und der Vorsprung 36, fixiert auf der oberen Elektrodenfläche 20-2, in Anlage mit der unteren Elektrodenfläche 20-4, bzw. dem Isolationsplättchen an der unteren Elektrode. Somit ist jede Elektrodenfläche maximal mit nur einem fixen Vorsprung versehen, wobei darüber hinaus zwei Elektrodenflächen überhaupt keinen fixen Vorsprung aufweisen.

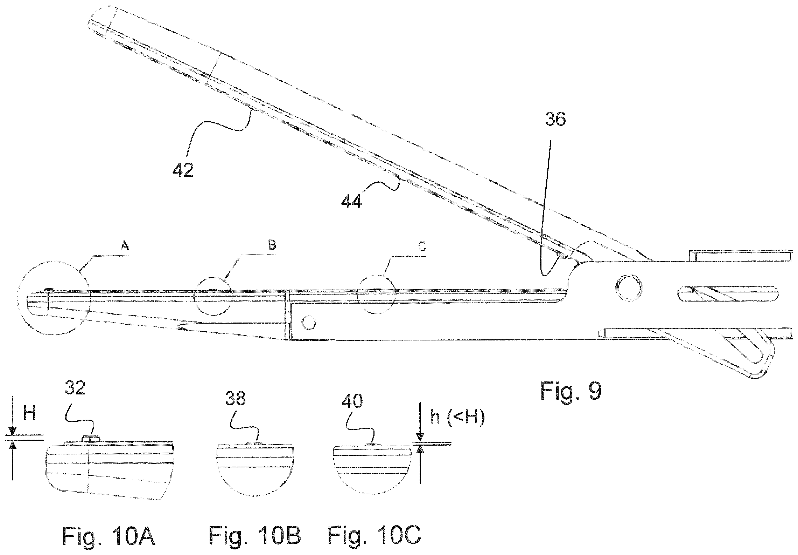

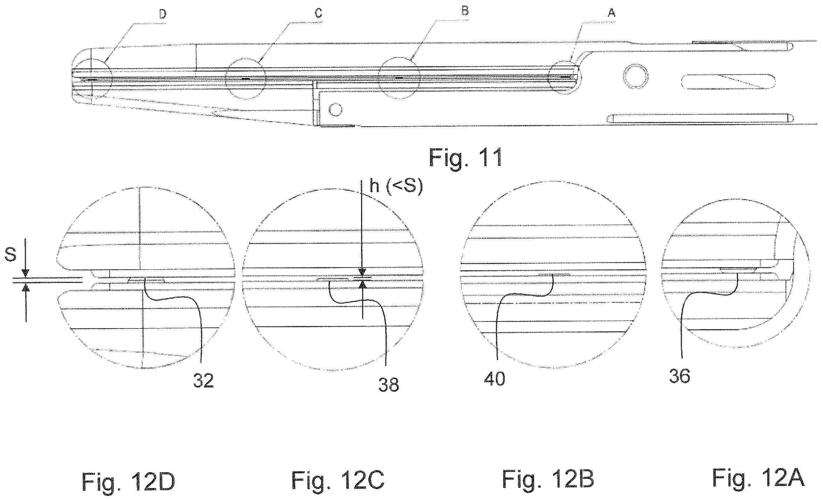

Aus der Fig. 8, insbesondere jedoch aus den weiteren Figuren 9 bis 12 ist zu erkennen, dass neben den als Abstandshalter dienenden Vorsprüngen 32 und 36 beispielsweise auf den Elektrodenflächen 20-3 und 20-2 jeweils zwei (oder eine andere Zahl größer null) Erhöhungen 38 und 40 bzw. 42 und 44 im medialen Bereich der Instrumentenbranchen 4 und 6 angeordnet sind. Aus den vergrößerten Ansichten 9A, 9B und 9C ist zu erkennen, dass die vorzugsweise noppenförmigen Erhöhungen 38 und 40 eine geringere Höhe als der Vorsprung 32 dieser Branche 6 aufweisen. Gleiches gilt für die Erhöhungen 42 und 44 bzw. Vorsprung 36 auf der oberen Instrumentenbranche 4. Wenn sich die Instrumentenbranchen 4 und 6 schließen, kommen somit lediglich die Vorsprünge 32 und 36 mit der jeweils gegenüberliegenden Elektrodenfläche 20-1 bzw. 20-4 in Anlage (siehe Fig. 11D und 11A), während die Erhöhungen 38, 40, 42, 44 von den gegenüberliegenden Elektrodenflächen beabstandet bleiben. Die Erhöhungen 38, 40, 42, 44 fungieren demnach nicht als Abstandshalter, sondern lediglich als Greifelemente, um ein Durchrutschen des zwischen den Branchen eingeklemmten Gewebes zu verhindern.

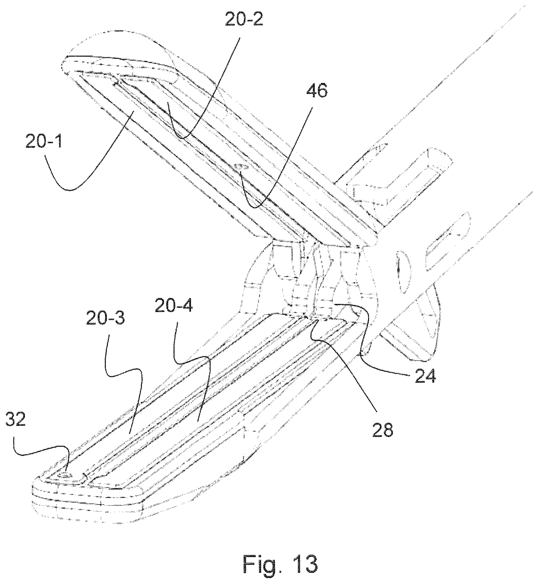

Fig. 13 zeigt eine vierte Ausführungsform, die sich von dem laparoskopischen elektrochirurgischen Instrument 2 der ersten Ausführungsform zum einen in der Anordnung des distalen Abstandshalters unterscheidet und zum anderen einen dritten medial angeordneten Abstandshalter aufweist. Anstatt zwischen den Elektrodenflächen 20-3 und 20-4 befindet sich als distaler Abstandshalter ein (noppenförmiger) Vorsprung 32 unmittelbar (d.h. fixiert) auf der Elektrodenfläche 20-3. Der proximale Abstandshalter wird wiederum durch das Abstandshaltermodul 24 gebildet, wie es anhand des ersten Ausführungsbeispiels bereits beschrieben wurde. Zusätzlich befindet sich (fixiert) auf der Elektrodenfläche 20-2 ein medial angeordneter (noppenförmiger) Vorsprung 46 mit der gleichen Höhe wie der Vorsprung 32 und die Materialzunge(n) 28. Dieser zusätzliche mediale Vorsprung wirkt einem (konvexen) Durchbiegen der Branchen und damit einem sich mittigen Annähern der Elektrodenflächen entgegen.

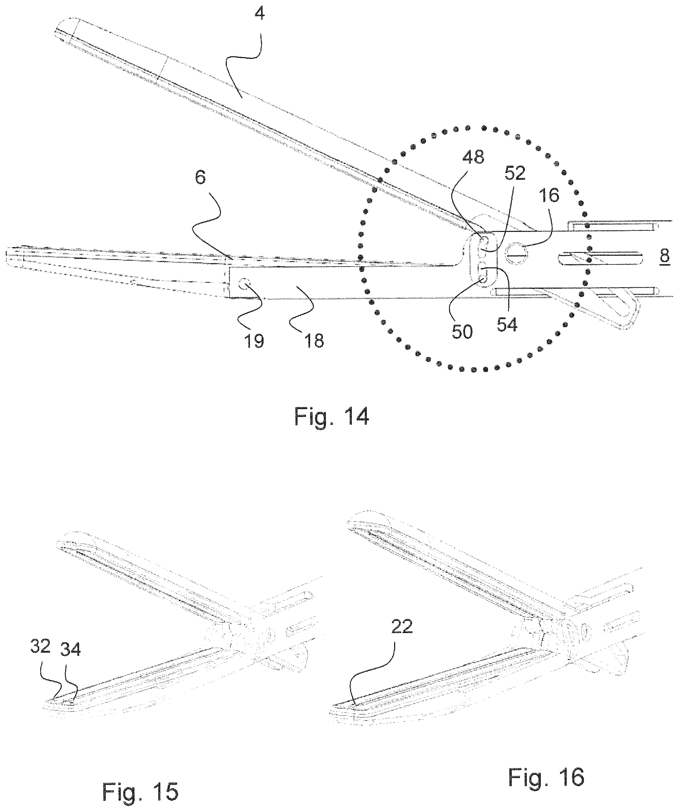

Fig. 14 zeigt eine fünfte Ausführungsform, die sich von dem laparoskopischen elektrochirurgischen Instrument 2 der ersten Ausführungsform lediglich in der Realisierung des auf die proximalen Abschnitte der Instrumentenbranchen 4 und 6 wirkenden Abstandhalters unterscheidet. Anstelle eines modularen Abstandshalters 24 weisen in diesem Fall die Instrumentenbranchen oder zumindest die eine betätigbare Branche an ihren proximalen Abschnitten (Endabschnitten) in Querrichtung der Branche sich erstreckende Drehbegrenzungsstifte 48 und 50 auf, die in rillenförmigen Kulissen 52 und 54 an der jeweils anderen (vorzugsweise unbetätigten) Branche bzw. an den Seitenwänden des schaftseitigen Durchgangs-Längsschlitzes aufgenommen und darin geführt sind. Die Kulissen 52 und 54 begrenzen den Freiheitsgrad der Drehbegrenzungsstifte 48 und 50 und somit den Schwenkbereich der zumindest einen Instrumentenbranche 4 (und/oder 6). Wenn die Drehbegrenzungsstifte 48 und 50 das jeweilige Ende der Kulissen 52 bzw. 54 erreichen, haben die Instrumentenbranchen 4 und 6 ihren vorbestimmten und gewünschten maximalen Öffnungs- und/oder Schließ-Abstand zueinander eingenommen. Das Zusammenwirken der Drehbegrenzungsstifte 48 und 50 und der Kulissen 52 und 54 dient/simuliert somit als proximaler Abstandshalter in dieser fünften Ausführungsform.

Fig. 15 zeigt eine perspektivische Ansicht des (geöffneten) Maulteils gemäß der fünften Ausführungsform, aus welcher zu erkennen ist, dass der distale Abstandshalter wie bei der zweiten Ausführungsform durch zwei (noppenförmige) Vorsprünge 32, 34 auf den Elektrodenflächen 20-3 und 20-4 der unteren Instrumentenbranche 6 (fixiert) gebildet wird.

Fig. 16 zeigt das (geöffnete Maulteil) gemäß einer sechsten Ausführungsform der Erfindung, die sich von dem laparoskopischen elektrochirurgischen Instrument 2 der fünften Ausführungsform lediglich in der Anordnung des/der distalen Abstandshalter(s) unterscheidet. Der distale Abstandshalter ist wie in der ersten Ausführungsform als ein zwischen den Elektrodenflächen 20-3 und 20-4 der unteren Instrumentenbranche 6 angeordneten (noppenförmiger) Vorsprung 22 umgesetzt. Ansonsten entspricht die sechste Ausführungsform der oben beschriebenen fünften Ausführungsform.

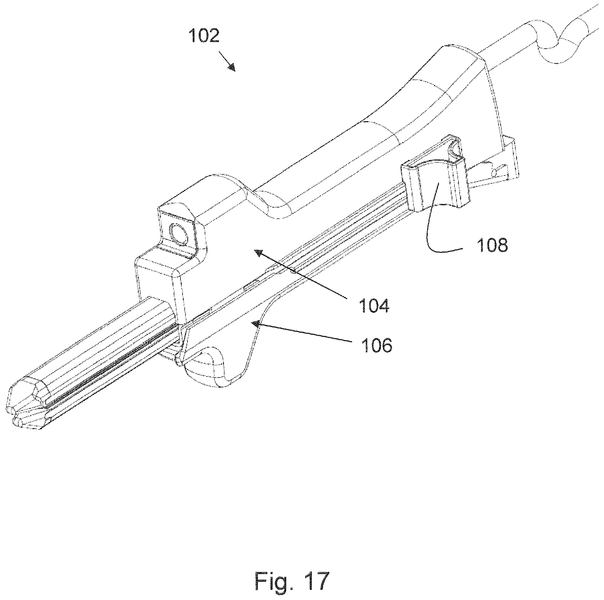

Fig. 17 zeigt ein elektrochirurgischen Instrument 102 gemäß einer siebten Ausführungsform, welches sich von dem oben beschriebenen Instrument 2 im Instrumententyp, insbesondere in der Bauart der Betätigungseinrichtung sowie der Handhabe unterscheidet. Während die vorstehend beschriebenen verschiedenen Ausführungsformen allesamt mit Bezug auf das in Fig. 1 dargestellte laparoskopische elektrochirurgische Instrument 2 mit einem dünnen, langen Schaft 12 und daran schwenkbar angelenkten Instrumentenbranchen 4 und 6 als distales Maulteil beschrieben wurde, sind die zuvor beschriebenen Varianten von Abstandshalteranordnungen ebenfalls im Zusammenhang mit dem Instrument 102 realisierbar, bei dem jedoch zwei Instrumentenbranchen 104 und 106 über ein Schieberelement 108 und einer entsprechenden (nicht weiter gezeigten) Mimik miteinander verbunden sind. Die Handhabe bzw. das Griffstück hat dabei eine Art Aufnahmeschacht, aus der die Instrumentenbranchen axial sowie distal herausragen. Dieser Schacht ist so dimensioniert, dass die Instrumentenbranchen bei deren Zurückziehen über das Schiebeelement 108 in den Schacht von diesem zusammengedrückt werden. Wird das Schiebeelement 108 wieder in Richtung Schachtöffnung vorgeschoben, bewegen sich die Instrumentenbranchen aus dem Schacht heraus und öffnen sich dabei vorzugsweise selbsttätig beispielsweise infolge einer Federvorspannung.

An dieser Stelle sei darauf hingewiesen, dass die vorliegende Erfindung nicht auf die zuvor beschriebene Ausführungsformen beschränkt ist. Diverse Modifikationen innerhalb des Schutzbereichs der beigefügten Ansprüche sind möglich.

So kann beispielsweise in der dritten Ausführungsform anstelle des Vorsprungs 36 auf der Elektrodenfläche 20-2 ein Abstandshaltermodul wie bei der ersten oder zweiten Ausführungsform als proximaler Abstandshalter verwendet werden.

Ferner können bei sämtlichen Ausführungsformen die Vorsprünge bzw. Erhöhungen auf anderen Elektrodenflächen angeordnet werden, solange auf jeder Elektrodenfläche gemäß dem vorliegenden bevorzugten Ausführungsbeispiel nur maximal ein Vorsprung aufgebracht (fixiert) ist.

Ferner sind Form und Größe der Anstandshalter variierbar, solange alle Abstandshalter so aufeinander abgestimmt sind, dass der Abstand zwischen den Elektrodenflächen an allen Stellen immer gleich ist. So kann der Abstandshalter beispielsweise auch pyramiden-(stumpf)-, zylinder- oder würfelförmig sein. Das Abstandshaltermodul kann schließlich in mehrere nebeneinander angeordnete Einzelmodule mit jeweils nur einer Materialzunge aufgeteilt werden.

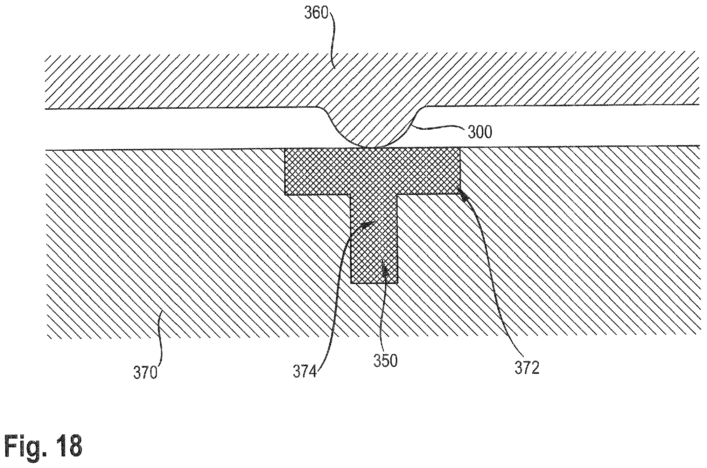

Nachfolgend werden ein Abstandshalter 300 sowie eine Abstandshalter-Aufstandsfläche 350 anhand der Fig. 18 bis 20 näher beschrieben, wie sie bei allen vorstehenden Ausführungsbeispielen eines Maulteils vorzugsweise auf der Elektrode grundsätzlich verwendet werden.

Wie bereits angeführt wurde, sind die Abstandshalter oder Vorsprünge 300 gemäß der vorliegenden Erfindung aus einem elektrisch leitenden Material (d.h. elektrisch leitend) vorzugsweise einstückig mit einer beispielhaft dargestellten, zugehörigen Elektrode 360 ausgebildet/verbunden. Der jeweilige Vorsprung 300 kann durch entsprechendes Ausstanzen und Biegen oder durch (punktuelles) Prägen/Pressen aus der Elektrode 360 selbst gefertigt sein. Es besteht prinzipiell aber auch die Möglichkeit, den elektrisch leitenden Vorsprung 300 beispielsweise in Form eines Kegels gemäß der Fig. 18 bis 20 oder einer Halbkugel auf der Elektrode 360 anzuschweißen, anzulöten oder anzuformen. Auf diese Weise wird in jedem Fall eine hohe Festigkeit zwischen Vorsprung 300 und Elektrode 360 sowie ein starrer Vorsprung selbst erzeugt, sodass ein unbeabsichtigtes Abkratzen oder Abbrechen des so ausgeformten Vorsprungs weitestgehend vermieden wird.

Auf einer gegenüberliegenden Elektrode 370 (der jeweils anderen Branche) sind im Bereich der Vorsprungs 300 teller- oder scheibenförmige Ausnehmungen oder Vertiefungen 372 ausgebildet. Diese Vertiefungen 372 besitzen zudem eine zentrale Sack- oder Durchgangsbohrung 374 in der Elektrode 370, die sich in Dickenrichtung der Elektrode 370 erstreckt. Auf diese Weise entsteht im Längsschnitt gemäß der Fig. 18 bis 20 eine Art pilzförmige Ausnehmung in der betreffenden Elektrode 370.

In diese Vertiefung ist ein Pin / Pad oder Stopfen 350 aus elektrisch nicht leitendem Material eingesetzt, dessen Form der Vertiefung im Wesentlichen exakt angepasst ist und der die Abstandshalter-Aufstandsfläche definiert. Alternativ hierzu ist es auch möglich, in die Vertiefung eine Vergussmasse aus elektrisch nicht leitendem Material einzuspritzen, die hierauf aushärtet.

Gemäß der Fig. 18 schließt der Stopfen 350 im Wesentlichen flächig bzw. eben mit der Oberfläche der betreffenden Elektrode 370 ab. Auf diese Weise bildet der Stopfen 350 keinen äußeren Angriffspunkt, um ggf. aus der Vertiefung herausgezogen werden zu können. Des Weiteren ist die äußere Fläche des Stopfens 350 im Wesentlichen auf den gegenüberliegenden Vorsprung 300 abgestimmt und nur so groß, dass der Vorsprung 300 bei Zusammenpressen der beiden Branchen sicher auf dem Stopfen 350 aufsitzt, ohne die betreffende Elektrode direkt zu berühren.

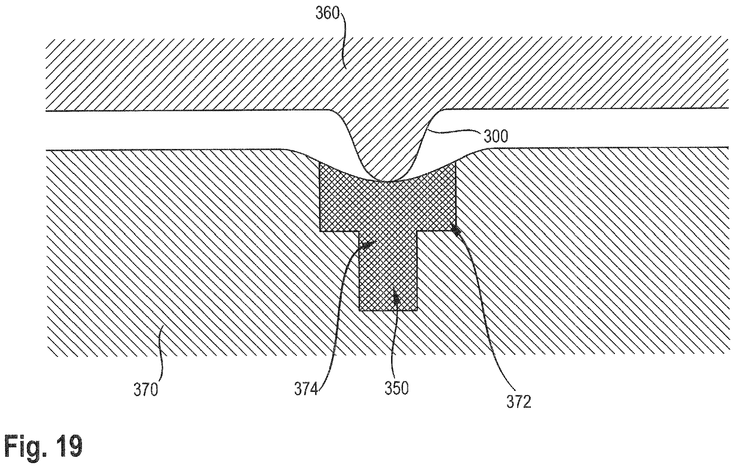

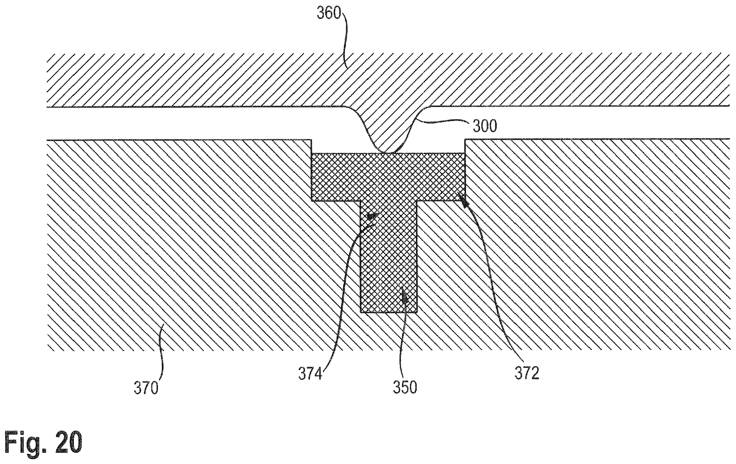

Die Fig. 19 und 20 zeigen jeweils alternative Ausgestaltungen für den Stopfen 350 (Abstandshalter-Aufstandsfläche), wonach gemäß der Fig. 19 eine konkave Außenfläche am Stopfen 350 vorgesehen ist, die ein besseres Zentrieren des gegenüberliegenden Vorsprungs 300 beim Zusammenpressen der Branchen bewirkt oder wonach gemäß der Fig. 20 der Stopfen 350 gegenüber der Oberfläche der betreffenden Elektrode (geringfügig) zurückgesetzt ist, um einen Überstand in jedem Fall zu vermeiden.

Als Material für die Abstandshalter-Aufstandsfläche bietet sich eine Keramik oder ein Kunststoff an. Auch kann zwischen der Abstandshalter-Aufstandsfläche (insbesondere dem Stopfen 350) und der Elektrode 370 eine Zwischenschicht vorgesehen sein, die hitzebedingte unterschiedliche Materialausdehnungen zwischen der Elektrode 370 und der Abstandshalter-Aufstandsfläche ausgleicht und so ein Zerbrechen oder Herausquellen der Abstandshalter-Aufstandsfläche aus der Ausnehmung 372 verhindert.

## Patentansprüche

1. Elektrochirurgisches Instrument (2) mit einem Maulteil aus gegeneinander bewegbaren Instrumentenbranchen (4, 6), an deren einander zugewandte Seiten jeweils eine oder mehrere Elektrodenflächen (20) angeordnet/ausgebildet sind, wobei die Bewegung der Instrumentenbranchen (4, 6) relativ zueinander durch zumindest einen auf proximale Endabschnitte der Instrumentenbranchen (4, 6) wirkenden ersten Abstandshalter (24; 36; 48, 50, 52, 54) und zumindest einen auf distale Endabschnitte der Instrumentenbranchen (4, 6) wirkenden zweiten Abstandshalter (22; 32, 34; 32) begrenzbar ist, wobei zumindest einer der Abstandshalter (300) an zumindest einer Elektrode (360) aus einem elektrisch leitenden Material gefertigt sowie elektrisch leitend mit der Elektrode (300) verbunden ist und mit einer lokalen Abstandshalter-Aufstandsfläche (350) aus einem nicht leitenden Material zusammenwirkt, die in elektrisch isolierender Weise auf zumindest einer gegenüberliegenden Elektrode (370) angeordnet ist,
**dadurch gekennzeichnet, dass**
die die Abstandshalter-Aufstandsfläche (350) aufweisende Elektrode (370) im Bereich des Abstandshalters (300) mit zumindest einer Vertiefung (372, 374) an ihrer Oberfläche ausgebildet ist, welche im Längsschnitt pilz- oder T-förmig ist, und wobei in diese Vertiefung (372, 374) ein nicht leitendes Material eingesetzt ist oder diese Vertiefung (372, 374) mit einem nicht leitenden Material ausgespritzt ist, welches die Abstandshalter-Aufstandsfläche (350) definiert.

2. Elektrochirurgisches Instrument (2) nach Anspruch 1, **dadurch gekennzeichnet, dass** die Abstandshalter-Aufstandsfläche (350) aus einem pad- oder pin-förmigen Bauteil besteht, deren Flächenweite in der Elektrodenebene über den Abstandshalter (300) allseitig vorragt, sodass der Abstandshalter (300) keinen elektrischen Kontakt mit der die Abstandshalter-Aufstandsfläche (250) aufweisenden Elektrode (370) erhält.

3. Elektrochirurgisches Instrument (2) nach einem der vorstehenden Ansprüche, **dadurch gekennzeichnet, dass** die die Abstandshalter-Aufstandsfläche (350) aufweisende Elektrode (370) mit zumindest einer Vertiefung (372) an ihrer Oberfläche ausgebildet ist, in welche die Abstandshalter-Aufstandsfläche (350) in Form eines Pad oder Pin eingesetzt ist oder welche mit einem elektrisch isolierenden Material ausgegossen ist, welches nach Erstarren die Abstandshalter-Aufstandsfläche (350) bildet.

4. Elektrochirurgisches Instrument (2) nach Anspruch 3, **dadurch gekennzeichnet, dass** die Abstandshalter-Aufstandsfläche (350) eben zur Elektrodenfläche ist, oder eine Konkavität aufweist oder bezüglich der Elektrodenfläche zurückgesetzt ist.

5. Elektrochirurgisches Instrument (2) nach einem der vorstehenden Ansprüche, **dadurch gekennzeichnet, dass** auf jeder Elektrodenfläche (20) maximal ein als elektrisch leitender Abstandshalter wirkender Vorsprung (32, 34; 32, 36; 32, 46) vorgesehen/fixiert ist.

6. Elektrochirurgisches Instrument (2) nach einem der vorhergehenden Ansprüche, **dadurch gekennzeichnet, dass**
zumindest ein Abstandshalter, insbesondere der erste Abstandshalter, durch ein separat von den Instrumentenbranchen (4, 6) ausgebildetes Abstandshaltermodul (24) gebildet ist, das zumindest eine elektrisch nicht leitende Materialzunge (28) aufweist, welche in einer Schließstellung zwischen den Instrumentenbranchen (4, 6) eingeklemmt wird, wobei die Höhe (H) der Materialzunge (28) einem vorbestimmten Mindestabstand (S) zwischen den Instrumentenbranchen (4, 6) in geschlossener Stellung entspricht.

7. Elektrochirurgisches Instrument (2) nach Anspruch 6, **dadurch gekennzeichnet, dass**
das Abstandshaltermodul (24) drehbar in einem Schwenkgelenk (16) einer verschwenkbaren Instrumentenbranche (4) gelagert ist und dabei insbesondere von wangenförmigen Gelenkabschnitten der verschwenkbaren Instrumentenbranche (4) umgriffen wird, welche einen Aufnahmehohlraum für das Abstandshaltermodul (24) definieren.

8. Elektrochirurgisches Instrument (2) nach Anspruch 1
**dadurch gekennzeichnet, dass**
auf jeder Elektrodenfläche (20) maximal ein als Abstandshalter wirkender Vorsprung (32, 34; 32, 36; 32, 46) vorgesehen/fixiert ist, wobei zumindest ein Abstandshalter, insbesondere der erste Abstandshalter, durch ein separat von den Instrumentenbranchen (4, 6) ausgebildetes Abstandshaltermodul (24) gebildet ist, das zumindest eine elektrisch nicht leitende Materialzunge (28) aufweist, welche in einer Schließstellung zwischen den Instrumentenbranchen (4, 6) eingeklemmt wird, wobei eine Höhe (H) der Materialzunge (28) einem vorbestimmten Mindestabstand (S) zwischen den Instrumentenbranchen (4, 6) in geschlossener Stellung entspricht und wobei das Abstandshaltermodul (24) drehbar in einem Schwenkgelenk (16) einer verschwenkbaren Instrumentenbranche (4) gelagert ist und dabei insbesondere von wangenförmigen Gelenkabschnitten der verschwenkbaren Instrumentenbranche (4) umgriffen wird, welche einen Aufnahmehohlraum für das Abstandshaltermodul (24) definieren.

9. Elektrochirurgisches Instrument (2) nach Anspruch 8, **dadurch gekennzeichnet, dass**
die eine Elektrodenbranche (4) an deren proximalem Endabschnitt in einer Lageröffnung der anderen Elektrodenbranche (6) oder deren Trägerteil (18) schwenkgeführt ist, indem die eine Elektrodenbranche (4) an deren proximalem Endabschnitt von der anderen Elektrodenbranche (6) oder deren Trägerteil (18) an diametralen Seiten umgriffen wird; und
der Aufnahmehohlraum des Abstandshaltermoduls (24) in der einen schwenkgeführten Elektrodenbranche (4) ausgebildet ist, derart, dass die wangenförmigen Gelenkabschnitte der einen schwenkgeführten Elektrodenbranche (4) zwischen dem Abstandshaltermodul (24) und der anderen Elektrodenbranche (6) zu liegen kommen.

10. Elektrochirurgisches Instrument (2) nach einem der vorstehenden Ansprüche, **dadurch gekennzeichnet, dass**
die Abstandshalter (22, 24, 32, 34, 48, 50, 52, 54) ausschließlich außerhalb eines für die Behandlung des Gewebes vorgesehenen Bereichs angeordnet sind.

11. Elektrochirurgisches Instrument (2) nach einem der vorhergehenden Ansprüche 1 bis 6, **dadurch gekennzeichnet, dass**
zumindest eine der Instrumentenbranchen (4, 6) einen in einer Kulisse (52, 54) vorzugsweise auf Seiten der jeweils anderen Instrumentenbranche (6, 4) geführten Drehbegrenzungsstift (48, 50) aufweist, wobei das Zusammenwirken von Drehbegrenzungsstift (48, 50) und Kulisse (52, 54) einen Abstandhalter, insbesondere den ersten Abstandshalter, bildet oder simuliert und die Instrumentenbranchen (4, 6) zueinander einen vorbestimmten Mindestabstandabstand (S) einnehmen, wenn der Drehbegrenzungsstift (48, 50) ein Ende der Kulisse (52, 54) erreicht.

12. Elektrochirurgisches Instrument (2) nach einem der vorhergehenden Ansprüche, **dadurch gekennzeichnet, dass**
zwischen dem proximalen und dem distalen Endabschnitt zumindest einer Instrumentenbranche (4, 6) ein oder mehrere zur jeweils anderen Instrumentenbranche (6, 4) hinweisende Erhöhungen (38, 40, 42, 44), vorzugsweise in gleichmäßigen Abständen, ausgebildet sind, deren Höhe (h) geringer als die Höhe (H) der Abstandshalter (24, 32, 34, 36, 46; 48, 50, 52, 54) ist und vorzugsweise 10% bis 75% der Höhe (H) der Abstandhalter (24, 32, 34, 36, 46; 48, 50, 52, 54) beträgt.

13. Elektrochirurgisches Instrument (2) nach Anspruch 8, **dadurch gekennzeichnet, dass**
ein Abstandshalter, insbesondere der zweite Abstandhalter, durch einen an einem distalen Endabschnitt einer Instrumentenbranche (6) zwischen zwei Elektrodenflächen (20-3, 20-4) dieser Instrumentenbranche (6) angeordneten und zur anderen Instrumentenbranche (4) hin weisenden Vorsprung (22) gebildet ist, oder
ein Abstandshalter, insbesondere der zweite Abstandhalter, durch einen auf einer Elektrodenfläche (20-3) oder durch mehrere auf jeweils unterschiedlichen Elektrodenflächen (20-3, 20-4) vorgesehene Vorsprünge (32; 32, 34) gebildet ist.

14. Elektrochirurgisches Instrument (2) nach Anspruch 8, **gekennzeichnet durch**
zumindest einen dritten Abstandshalter, der auf mediale Abschnitte der Instrumentenbranchen (4, 6) wirkt, wobei der dritte Abstandshalter insbesondere als ein Vorsprung ausgebildet und so angeordnet ist, dass die Anzahl an auf einer Elektrodenfläche (20-2) angeordneten/fixierten Abstandshaltern den Wert 1 nicht übersteigt.

## Claims

1. An electrosurgical instrument (2) comprising a jaw part made up of mutually movable instrument legs or branches (4, 6) which have facing sides on which one or more electrode surfaces (20) are arranged/formed in each case, the movement of the instrument legs or branches (4, 6) relative to each other being able to be limited by at least one first spacer (24; 36; 48, 50, 52, 54) acting on proximal end portions of the instrument legs or branches (4, 6) and at least one second spacer (22; 32, 34; 32) acting on distal end portions of the instrument legs or branches (4, 6),
wherein at least one of the spacers (300) on at least one electrode (360) is manufactured from an electrically conductive material and is connected to the electrode (300) in electroconductive fashion, and cooperates with a local spacer abutment surface (350) which is made of a non-conductive material and arranged in electrically insulating manner on at least one opposing electrode (370),
**characterized in that**
the electrode (370) comprising the spacer abutment surface (350) is formed to have at least one indentation (372, 374) on its surface in the region of the spacer (300), wherein the indentation (372, 374) is mushroom-shaped or T-shaped as seen in longitudinal section, and wherein an electrically non-conductive material is inserted into this indentation (372, 374) or this indentation (372, 374) is injected with an electrically non-conductive material defining the spacer abutment surface (350).

2. The electrosurgical instrument (2) according to claim 1, **characterized in that the** spacer abutment surface (350) consists of a pad-shaped or pin-shaped component whose areal width on the electrode plane protrudes beyond the spacer (300) on all sides, so that the spacer (300) does not come into electrical contact with the electrode (370) comprising the spacer abutment surface (250).

3. The electrosurgical instrument (2) according to one the preceding claims, **characterized in that** the electrode (370) comprising the spacer abutment surface (350) is formed to have at least one indentation (372) on its surface, in which the spacer abutment surface (350) in the form of a pad or pin is inserted, or which is cast with an electrically insulating material which forms the spacer abutment surface (350) after solidification.

4. The electrosurgical instrument (2) according to claim 3, **characterized in that** the spacer abutment surface (350) is level with the electrode area, or comprises a concavity, or is recessed with respect to the electrode area,

5. The electrosurgical instrument (2) according to one of the preceding claims, **characterized in that** at most one protrusion (32, 34; 32, 36; 32, 46) acting as an electrically conductive spacer is provided/fixed on each of the electrode areas (20).

6. The electrosurgical instrument (2) according to one of the preceding claims, **characterized in that**
at least one spacer, in particular the first spacer, is formed by a spacer module (24) which is formed to be separate from the instrument legs or branches (4, 6) and comprises at least one electrically non-conductive material tongue (28) which is clamped between the instrument legs or branches (4, 6) in a closed position, the height (H) of the material tongue (28) corresponding to a predetermined minimum distance (S) between the instrument legs or branches (4, 6) in the closed position.

7. The electrosurgical instrument (2) according to claim 6, **characterized in that** the spacer module (24) is rotatably supported in a swivel joint (16) of a pivotable instrument leg or branch (4) and is enclosed in particular by flange-shaped joint portions of the pivotable instrument leg or branch (4), which define an accommodation cavity for the spacer module (24).

8. The electrosurgical instrument (2) according to claim 1,
**characterized in that**
at most one protrusion (32, 34; 32, 36; 32, 46) acting as a spacer is provided/fixed on each of the electrode areas (20), at least one spacer, in particular the first spacer, being formed by a spacer module (24) which is formed to be separate from the instrument legs or branches (4, 6) and comprises at least one electrically non-conductive material tongue (28) which is clamped between the instrument legs or branches (4, 6) in a closed position, a height (H) of the material tongue (28) corresponding to a predetermined minimum distance (S) between the instrument legs or branches (4, 6) in the closed position, and the spacer module (24) being rotatably supported in a swivel joint (16) of a pivotable instrument leg or branch (4) and being enclosed in particular by flange-shaped joint portions of the pivotable instrument leg or branch (4), which define an accommodation cavity for the spacer module (24).

9. The electrosurgical instrument (2) according to claim 8, **characterized in that** the one instrument leg or branch (4) is pivotally guided at its proximal end portion in a mounting opening of the other instrument leg or branch (6) or its carrier part (18), by said one instrument leg or branch (4) having its proximal end portion enclosed by the other instrument leg or branch (6) or its carrier part (18) at diametric sides; and
the accommodation cavity of the spacer module (24) being formed in the one instrument leg or branch (4), which is pivotally guided, in such a way that the flange-shaped joint portions of said pivotally guided instrument leg or branch (4) come to lie between the spacer module (24) and the other instrument leg or branch (6).

10. The electrosurgical instrument (2) according to one of the preceding claims, **characterized in that**
the spacers (22, 24, 32, 34, 48, 50, 52, 54) are arranged exclusively outside a region provided for tissue treatment.

11. The electrosurgical instrument (2) according to one of the preceding claims 1 to 6, **characterized in that**
at least one of the instrument legs or branches (4, 6) comprises a rotation limiter pin (48, 50) which is guided in a slot-type guide (52, 54) preferably on the side of the respective other instrument leg or branch (6, 4), the cooperation between the rotation limiter pin (48, 50) and the slot-type guide (52, 54) establishing or simulating a spacer, in particular the first spacer, and the instrument legs or branches (4, 6) having a predetermined minimum distance (S) relative to each other if the rotation limiter pin (48, 50) reaches an end of the slot-type guide (52, 54).

12. The electrosurgical instrument (2) according to one of the preceding claims, **characterized in that**
between the proximal end portion and the distal end portion of at least one instrument leg or branch (4, 6), one or more ridges (38, 40, 42, 44) pointing toward the respective other instrument leg or branch (6, 4) are formed preferably at regular intervals, said ridges having a height (h) which is less than the height (H) of the spacers (24, 32, 34, 36, 46; 48, 50, 52, 54) and preferably amounts to 10% to 75% of the height (H) of the spacers (24, 32, 34, 36, 46; 48, 50, 52, 54).

13. The electrosurgical instrument (2) according to claim 8, **characterized in that** a spacer, in particular the second spacer, is formed by a protrusion (22) which is arranged on a distal end portion of an instrument leg or branch (6) between two electrode areas (20-3, 20-4) of said instrument leg or branch (6) and points toward the other instrument leg or branch (4), or
a spacer, in particular the second spacer, is formed by a protrusion (32; 32, 34) provided on an electrode area (20-3) or by several protrusions (32; 32, 34) each provided on different electrode areas (20-3, 20-4).

14. The electrosurgical instrument (2) according to claim 8, **characterized by**
at least one third spacer which acts on middle portions of the instrument legs or branches (4, 6), said third spacer being formed in particular as a protrusion and being arranged such that the number of spacers which are arranged/fixed on one electrode area (20-2) does not exceed the value 1.

## Revendications

1. Instrument chirurgical électrique (2) avec une mâchoire composée de branches d'instrument (4, 6) qui sont mobiles l'une par rapport à l'autre et au niveau des côtés proches desquelles sont agencées/réalisées à chaque fois une ou plusieurs surfaces d'électrode (20), dans lequel le mouvement des branches d'instrument (4, 6) l'une par rapport à l'autre peut être limité par au moins un premier écarteur (24 ; 36 ; 48, 50, 52, 54) agissant sur des extrémités proximales des branches d'instrument (4, 6) et au moins un deuxième écarteur (22 ; 32, 34 ; 32) agissant sur des extrémités distales des branches d'instrument (4, 6), dans lequel au moins l'un des écarteurs (300) au niveau d'au moins une électrode (360) est fabriqué en un matériau électriquement conducteur et est relié de manière électriquement conductrice à l'électrode (300) et coopère avec une surface de contact d'écarteur (350) locale qui est en un matériau non conducteur et qui est agencée de manière électriquement isolante sur au moins une électrode opposée (370),
**caractérisé en ce que**
l'électrode (370) comportant la surface de contact d'écarteur (350) est réalisée au niveau de sa surface et dans la zone de l'écarteur (300) avec au moins un creux (372, 374) qui est en forme de champignon ou de T en coupe longitudinale, et dans lequel un matériau non conducteur est placé dans ce creux (372, 374) ou ce creux (372, 374) est réalisé par injection avec un matériau non conducteur qui définit la surface de contact d'écarteur (350).

2. Instrument chirurgical électrique (2) selon la revendication 1, **caractérisé en ce que** la surface de contact d'écarteur (350) est constituée d'un élément formant une broche ou un ergot dont la largeur de surface dépasse de tous côtés dans le plan d'électrode au-delà de l'écarteur (300) de telle sorte que l'écarteur (300) n'a pas de contact électrique avec l'électrode (370) comportant la surface de contact d'écarteur (250).

3. Instrument chirurgical électrique (2) selon l'une quelconque des revendications précédentes, **caractérisé en ce que** l'électrode (370) comportant la surface de contact d'écarteur (350) est réalisée au niveau de sa surface avec au moins un creux (372) dans lequel est placée la surface de contact d'écarteur (350) sous la forme d'un ergot ou d'une broche ou qui est rempli avec un matériau électriquement isolant qui forme après solidification la surface de contact d'écarteur (350).

4. Instrument chirurgical électrique (2) selon la revendication 3, **caractérisé en ce que** la surface de contact d'écarteur (350) est plane par rapport à la surface d'électrode ou présente une concavité ou est en retrait par rapport à la surface d'électrode.

5. Instrument chirurgical électrique (2) selon l'une quelconque des revendications précédentes, **caractérisé en ce qu'**au maximum une partie en saillie (32, 34; 32, 36; 32, 46) agissant comme écarteur électriquement conducteur est prévue/fixée sur chaque surface d'électrode (20).

6. Instrument chirurgical électrique (2) selon l'une quelconque des revendications précédentes, **caractérisé en ce que**
au moins un écarteur, en particulier le premier écarteur, est formé par un module écarteur (24) qui est réalisé séparément des branches d'instrument (4, 6) et qui comporte au moins une langue de matériau (28) électriquement non conductrice qui est serrée entre les branches d'instrument (4, 6) dans une position fermée, la hauteur (H) de la langue de matériau (28) correspondant à une distance minimale prédéterminée (S) entre les branches d'instrument (4, 6) en position fermée.

7. Instrument chirurgical électrique (2) selon la revendication 6, **caractérisé en ce que**
le module écarteur (24) est logé de manière à pouvoir tourner dans une articulation pivotante (16) d'une branche d'instrument (4) pivotante et est ainsi entouré en particulier par des parties d'articulation en forme de joues de la branche d'instrument (4) pivotante qui définissent une cavité de logement pour le module écarteur (24).

8. Instrument chirurgical électrique (2) selon la revendication 1,
**caractérisé en ce que**
au maximum une partie en saillie (32, 34; 32, 36; 32, 46) agissant comme écarteur est prévue/fixée sur chaque surface d'électrode (20), dans lequel au moins un écarteur, en particulier le premier écarteur, est formé par un module écarteur (24) qui est réalisé séparément des branches d'instrument (4, 6) et qui comporte au moins une langue de matériau (28) électriquement non conductrice qui est serrée entre les branches d'instruments (4, 6) dans une position fermée, dans lequel une hauteur (H) de la langue de matériau (28) correspond à une distance minimale prédéterminée (S) entre les branches d'instrument (4, 6) en position fermée et dans lequel le module écarteur (24) est logé de manière à pouvoir tourner dans une articulation pivotante (16) d'une branche d'instrument (4) pivotante et est ainsi entouré en particulier par des parties d'articulation en forme de joues de la branche d'instrument (4) pivotante qui définissent une cavité de logement pour le module écarteur (24).

9. Instrument chirurgical électrique (2) selon la revendication 8, **caractérisé en ce que**
la branche d'électrode (4) est guidée pour le pivotement au niveau de son extrémité proximale dans une ouverture de support de l'autre branche d'électrode (6) ou de la partie porteuse (18) de celle-ci, la branche d'électrode (4) étant entourée au niveau de son extrémité proximale par l'autre branche d'électrode (6) ou par la partie porteuse (18) de celle-ci au niveau des côtés diamétralement opposés ; et
la cavité de logement du module écarteur (24) est réalisée dans la branche d'électrode (4) guidée en pivotement de telle sorte que les parties articulées en forme de joues de la branche d'électrode (4) guidée en pivotement viennent se placer entre le module écarteur (24) et l'autre branche d'électrode (6).

10. Instrument chirurgical électrique (2) selon l'une quelconque des revendications précédentes, **caractérisé en ce que**
les écarteurs (22, 24, 32, 34, 48, 50, 52, 54) sont agencés exclusivement en dehors d'une zone prévue pour la manipulation du tissu.

11. Instrument chirurgical électrique (2) selon l'une quelconque des revendications 1 à 6, **caractérisé en ce que**
au moins une des branches d'instrument (4, 6) comporte une tige de limitation de rotation (48, 50) guidée dans une coulisse (52, 54) de préférence sur des côtés de l'autre branche d'instrument respective (6, 4), dans lequel la coopération de la tige de limitation de rotation (48, 50) et de la coulisse (52, 54) forme ou simule un écarteur, en particulier le premier écarteur, et les branches d'instrument (4, 6) prennent l'une par rapport à l'autre une distance minimale prédéterminée (S) lorsque la tige de limitation de rotation (48, 50) atteint une extrémité de la coulisse (52, 54).

12. Instrument chirurgical électrique (2) selon l'une quelconque des revendications précédentes, **caractérisé en ce que,**
entre l'extrémité proximale et l'extrémité distale d'au moins une branche d'instrument (4, 6), sont réalisées une ou plusieurs bosses (38, 40, 42, 44) pointant vers l'autre branche d'instrument respective (6, 4), de préférence à intervalles réguliers, bosses dont la hauteur (h) est inférieure à la hauteur (H) des écarteurs (24, 32, 34, 36, 46 ; 48, 50, 52, 54) et est égale de préférence à 10 % à 75 % de la hauteur (H) des écarteurs (24, 32, 34, 36, 46 ; 48, 50, 52, 54).

13. Instrument chirurgical électrique (2) selon la revendication 8, **caractérisé en ce que**
un écarteur, en particulier le deuxième écarteur, est formé par une partie en saillie (22) agencée au niveau d'une extrémité distale d'une branche d'instrument (6) entre deux surfaces d'électrode (20-3, 20-4) de cette branche d'électrode (6) et pointant vers l'autre branche d'instrument (4), ou
un écarteur, en particulier le deuxième écarteur, est formé par une partie en saillie (32 ; 32, 34) prévue sur une surface d'électrode (20-3) ou sur des surfaces d'électrode (20-3, 20-4) respectivement différentes.

14. Instrument chirurgical électrique (2) selon la revendication 8, **caractérisé par**
au moins un troisième écarteur qui agit sur des parties médianes des branches d'instrument (4, 6), lequel troisième écarteur est réalisé comme une partie en saillie et est agencé de telle sorte que le nombre d'écarteurs agencés/fixés sur une surface d'électrode (20-2) ne dépasse pas la valeur 1.
